# EUROPEAN PATENT APPLICATION

(11) **EP 4 424 304 A1**
(43) Date of publication of application: **04.09.2024**
(21) Application number: 22892990.7
(22) Date of filing: 30.08.2022
(51) Int. Cl.: A61K 9/51, B01J 2/02, B01J 19/00

(54) **METHOD FOR PREPARING MEDICINES, HAVING REDUCED PRODUCTION TIME AND IMPROVED STABILITY**

(30) Priority: 12.11.2021 KR 20210155303
(71) Applicant: Dr.Inb Co., Daejeon 34047 (KR)
(72) Inventor: KIM, Seong U, Daejeon 34107 (KR); SONG, Young Kyu, Daejeon 35217 (KR); SHIM, Young Key, Paju-si Gyeonggi-do 10909 (KR); LEE, Tae Heon, Sejong 30083 (KR); KIM, Hye Jeong, Daejeon 34177 (KR)
(74) Representative: WSL Patentanwälte Partnerschaft mbB
(86) International application number: PCT/KR2022/012937
(87) International publication number: WO 2023/085567

(57) **Abstract**

The present invention relates to: a novel drug preparation method capable of preparing medicines, nano-drug-carriers or metal-organic frameworks, which are prepared by means of a conventional wet chemical synthesis method; and a drug prepared by means of the preparation method.

## Description

### [Technical Field]

The present disclosure relates to a novel drug production method capable of producing medicines, nano-drug delivery systems, or metal-organic frameworks, which have been conventionally manufactured by wet chemical synthesis methods, and to a drug produced thereby.

### [Background Art]

Various methods for synthesizing nanomaterials using wet chemical synthesis methods have been studied. The basic parameters that affect the properties of nanomaterials include the concentration of reactants, pH of precursors, heating temperature, and reaction time.

Parameters for synthesizing high-quality nanoparticles as products include narrow particle size distribution, crystal quality, desired morphology of the product nanomaterial, sufficient supersaturation, careful selection of precursors, and concentration of stabilizer species. These synthesis parameters play a key role in long-term stability of nanoparticles. In order to mix precursors well at the atomic scale, methods based on this route enable preparation of materials with relatively good stoichiometry and sizes ranging from 1 nm to ones of microns, and easy stabilization using capping agents.

The categories of wet chemical synthesis methods developed to date are as follows.

First, there is chemical bath deposition synthesis, in which precipitation of a solid phase occurs due to supersaturation as a result of chemical reaction in a reaction solution. Additionally, there is successive ion layer adsorption and reaction (SILAR), which may be used to deposit a uniform film on any substrate with a controlled deposition rate, composition, and thickness and does not require vacuum and in which the process proceeds at low temperatures, obviating the need for expensive and complex technical equipment. There is also a chemical co-precipitation method, which allows nanoparticles to grow by dissolving and mixing precursors separately and then mixing the same with a precipitation reagent. Moreover, there is electrochemical synthesis, in which electric current is used as a driving force to deposit a nanocrystal thin film on a substrate in the presence of a substrate, two or three electrodes, an electrolyte solution in a vessel, and a current source. Furthermore, in order to synthesize nanoparticles in an organic solvent, there are solvothermal synthesis, in which physicochemical reaction is carried out under conditions of high pressure and high temperature, and hydrothermal synthesis, in which reaction is carried out in an aqueous solution. There is also sol-gel synthesis, in which a liquid (sol) is chemically converted into a gel and then condensed into a solid nanostructure. In addition, there is microemulsion synthesis, in which reaction is carried out using a uniform solution including water, oil, a surfactant, and an amine-based or alcohol auxiliary surfactant. This technology includes a collection of processes in the thermodynamically established colloidal system, involving an isotropic mixture of hydrophilic liquid (water), lipophilic liquid (oil), and amphiphilic surfactant (hydrophilic and hydrophobic groups).

As reported, among these synthesis strategies, double-precursor synthesis or hydrothermal synthesis enables formation of Berlin blue nanoparticles and analogues thereof having a narrow size distribution, stable dispersion in a solution, and a uniform morphology. However, a double-precursor synthesis method is disadvantageous in that it is difficult to create a uniform morphology compared to hydrothermal synthesis and also control of the growth rate is difficult. Also, hydrothermal synthesis is problematic because a long time for synthesis is required, excess stabilizers are needed and are difficult to dissolve, and toxic substances are produced as byproducts. Gold nanoparticles are synthesized by hydrothermal synthesis, and are manufactured at high temperatures using sodium citrate (Na₃C₆H₅O₇) as a reducing agent and surfactant. However, due to dependence only on sodium citrate, it is easy for the reduced gold atoms to agglomerate immediately, which is undesirable. Moreover, it is not easy to manufacture particles having a uniform size in a short time, so there is a need to devise a method capable of finely controlling nanoparticles.

Existing methods of synthesizing nanoparticles require expensive equipment, have relatively low productivity per hour, and make manufacture of particles with uniform size distribution difficult. Furthermore, when the particles are manufactured, harmful reactive gases such as chlorine gas and byproducts may be generated, and due to the manufacture of particles in a molecular state, it is difficult to form nanoparticles composed of various materials, and the manufacturing conditions and processes are complicated. In particular, the particles may agglomerate due to the surface effect of nanoparticles during transport or final application of nanoparticles, and thus reprocessing nanoparticles through methods such as dispersion in the solution, regrinding, or ultrasonic treatment is additionally required, and in the case of metal, there are problems such as oxidation, etc. due to exposure.

### [Citation List]

### [Patent Literature]

(Patent Document 1) Korean Patent No. 10-1329646

### [Disclosure]

### [Technical Problem]

The present disclosure has been made keeping in mind the problems in the manufacture, storage, and application of organic or inorganic nanoparticles and chemical drugs as described above, and an object of the present disclosure is to provide a method of manufacturing organic or inorganic nanoparticles and a nanomedicine, in which the particles are uniform in size, agglomeration and oxidation reaction are minimized, the time required for synthesis is reduced, the yield is improved within the same reaction time, synthesis is possible even at high temperatures, and various types of particles are easily manufactured, and a method of producing a synthetic medicine and a biomedicine with increased product yield.

The objects of the present disclosure are not limited to the foregoing. The objects of the present disclosure will be able to be clearly understood through the following description and to be realized by the means described in the claims and combinations thereof.

### [Technical solution]

The present disclosure has the effect of shortening the overall synthesis time by designing a synthesis method in cut liquid to solve the time-consuming heat transfer during the synthesis process.

An aspect of the present disclosure provides a method of manufacturing nanoparticles or a nanomedicine, including (a) adding a synthesis material and a solvent to a precursor mixing chamber to which an injection pipe is connected and performing vortexing, (b-1) moving the solution prepared in step (a) to a reaction chamber 30 connected to the mixing chamber, and (b-2) allowing gas to flow at a high speed on the boundary of the reaction chamber 30 and in a liquid cutting tube 31 with a gap ranging from 10 nanometers to 1,000 micrometers therebetween.

In an aspect of the present disclosure, the method may further include (c) collecting the cut solution from the boundary of the reaction chamber 30 and the liquid cutting tube 31 and collecting the same again in a predetermined amount at the boundary of the reaction chamber.

In an aspect of the present disclosure, the method may further include (d) separating or extracting a synthesized reaction product from the boundary and performing circulation to bind a target material in a separate binding chamber.

In the method according to an aspect of the present disclosure, the side angle of the inlet of the liquid cutting tube 31 may be 5 degrees to 60 degrees, and the front angle may be 5 degrees to 60 degrees.

In the method according to an aspect of the present disclosure, in step (b-2), the gas may be designed to flow at a high speed due to voltage, and the gas may flow at a high speed at an applied voltage of 5 to 60 kV.

In the method according to an aspect of the present disclosure, the boundary of the reaction chamber and the liquid cutting tube may be at 0.25 to 0.85 rad.

In the method according to an aspect of the present disclosure, the gap in step (b-2) may be 500 nm to 1500 nm.

In the method according to an aspect of the present disclosure, the nanoparticles or the nanomedicine may be a photosensitizer, a first-generation anticancer agent, a second-generation anticancer agent, a third-generation anticancer agent, a nuclear medicine therapeutic drug, a metabolic anticancer agent, an enzyme, a gene therapeutic agent, or a near-infrared fluorescent dye.

Another aspect of the present disclosure provides nanoparticles or a nanomedicine manufactured by the method according to any one aspect of the present disclosure described above.

### [Advantageous effects]

A synthesis method of the present disclosure is designed to enable rapid synthesis of drugs by continuously converting bulk liquid into small cut liquid to speed up heat transfer, thereby facilitating dissolution of a poorly soluble material and increasing the synthesis rate of a compound.

According to the synthesis method in cut liquid depending on the gas flow rate of the present disclosure, the size of the particles is uniform, agglomeration and oxidation reaction are minimized, the time required for synthesis is shortened, the yield is improved within the same reaction time, synthesis is possible even at high temperatures, and it is easy to manufacture various types of particles through a simple manufacturing process, effectively producing and providing high-quality nanoparticles and chemical drugs economically.

The manufacturing method according to an aspect of the present disclosure can increase water solubility of the manufactured poorly water-soluble material, compared to existing methods.

The manufacturing method according to an aspect of the present disclosure can increase stability of the manufactured material, compared to existing methods.

The effects of the present disclosure are not limited to the foregoing. It should be understood that the effects of the present disclosure include all effects that can be inferred from the following description.

### [Description of Drawings]

FIG. 1 shows the concept of the present disclosure and the structure of a synthesis device;
FIG. 2 shows the structures of synthesized final products;
FIGs. 3a to 3c show results of the temperature rotation of the device and the time taken for a mixed solution to reach a target temperature;
FIGs. 4a to 4i show results of analysis of the effect of killing cancer cells;
FIG. 5 shows images of cancer cells subjected to phototherapy (photomedicine);
FIGs. 6a and 6b show results of the time taken for a cerium mixed solution to reach the target temperature;
FIGs. 7a and 7b show results of the time taken for a gold ion mixed solution to reach the target temperature;
FIGs. 8a and 8b show results of the time taken for a palladium mixed solution to reach the target temperature;
FIGs. 9a and 9b show purity and UV-Vis spectra of MPPa synthesized by the manufacturing method of the present disclosure;
FIG. 10 shows ¹H NMR analysis results of MPPa synthesized by the manufacturing method of the present disclosure;
FIGs. 11a and 11b show results of the time taken for a methyl pheophorbide-a mixed solution to reach the target temperature;
FIGs. 12a to 12c show results of the time taken for a docetaxel mixed solution to reach the target temperature;
FIGs. 13a and 13b show results of the time taken for a hypromellose mixed solution to reach the target temperature;
FIGs. 14a and 14b show results of UV-Vis spectra and dot blot analysis of an antibody-drug conjugate; and
FIGs. 15a and 15b show results of the time taken for a 6-maleimidocaproic acid mixed solution to reach the target temperature.

### [Best Mode]

The above and other objects, features and advantages of the present disclosure will be more clearly understood from the following preferred embodiments taken in conjunction with the accompanying drawings. However, the present disclosure is not limited to the embodiments disclosed herein, and may be modified into different forms. These embodiments are provided to thoroughly explain the disclosure and to sufficiently transfer the spirit of the present disclosure to those skilled in the art.

It will be understood that the terms "comprise", "include", "have", etc., when used in this specification, specify the presence of stated features, integers, steps, operations, elements, components, or combinations thereof, but do not preclude the presence or addition of one or more other features, integers, steps, operations, elements, components, or combinations thereof.

Unless otherwise specified, all numbers, values, and/or representations that express the amounts of components, reaction conditions, polymer compositions, and mixtures used herein are to be taken as approximations including various uncertainties affecting measurement that inherently occur in obtaining these values, among others, and thus should be understood to be modified by the term "about" in all cases. Furthermore, when a numerical range is disclosed in this specification, the range is continuous, and includes all values from the minimum value of said range to the maximum value thereof, unless otherwise indicated. Moreover, when such a range pertains to integer values, all integers including the minimum value to the maximum value are included, unless otherwise indicated.

In the present specification, when a range is described for a variable, it will be understood that the variable includes all values including the end points described within the stated range. For example, the range of "5 to 10" will be understood to include any subranges, such as 6 to 10, 7 to 10, 6 to 9, 7 to 9, and the like, as well as individual values of 5, 6, 7, 8, 9 and 10, and will also be understood to include any value between valid integers within the stated range, such as 5.5, 6.5, 7.5, 5.5 to 8.5, 6.5 to 9, and the like. Also, for example, the range of "10% to 30%" will be understood to include subranges, such as 10% to 15%, 12% to 18%, 20% to 30%, etc., as well as all integers including values of 10%, 11%, 12%, 13% and the like up to 30%, and will also be understood to include any value between valid integers within the stated range, such as 10.5%, 15.5%, 25.5%, and the like.

**Hereinafter, a detailed description will be given of the present disclosure.**

In the synthesis of compounds, heat is an important factor that facilitates melting and dissolution of chemicals and determines the rate of synthesis. Moreover, in the synthesis of drugs, the yield, amounts of byproducts, uniformity and the like vary depending on the heat transfer rate. Accordingly, manufacturers are trying to control heat in the synthesis process. One fact is that heat transfer is faster in liquid with a smaller volume than in bulk liquid (solution or mixed solution). Therapeutic agents needed for pandemic diseases such as COVID-19 require synthesis in bulk, but as the volume increases, problems arise with synthesis time, yield, and uniformity. However, manufacturing a lot of products in small units increases purification time and installation cost. As a way to solve this problem, the synthesis method of the present disclosure is designed to enable rapid synthesis of drugs by continuously converting bulk liquid into small cut liquid to speed up heat transfer and increase the rate of synthesis.

In addition, the present disclosure is devised by improving the synthesis method in cut liquid depending on the gas flow rate to solve the problems in the manufacture, storage, and application of organic or inorganic nanoparticles and chemical drugs as described above. The present disclosure is intended to provide a method of manufacturing organic or inorganic nanoparticles and a nanomedicine, in which particles are uniform in size, agglomeration and oxidation reaction are minimized, the time required for synthesis is shortened, the yield within the same reaction time is improved, synthesis even at high temperatures is possible, and it is easy to manufacture various types of particles, and a method of producing a synthetic medicine and a biomedicine with increased product yield.

The present disclosure pertains to a method of shortening the production time of chemicals and manufacturing medicines uniformly, and also to a nano-drug delivery system that increases water solubility of a drug and helps the drug to achieve intended efficacy thereof and a method of manufacturing the same. Metal-organic framework (MOF) nanoparticles that may be stabilized for a long time even in sterile aqueous solutions frequently used in biological or medical experiments are manufactured by a synthesis method in cut liquid depending on the gas flow rate that has not been conventionally used in wet chemical synthesis methods. Nanoparticles made by the manufacturing method of the present disclosure, compared to existing methods of manufacturing MOF nanoparticles, may increase water solubility of pyropheophorbide-a methyl ester, which is a poorly soluble photosensitizer, and the phototherapy effect on cancer cells is also increased. Additionally, the synthesis method of the present disclosure is effective at shortening the production time of drugs made through existing chemical synthesis and biomedicines made through binding.

The method of manufacturing nanoparticles or a medicine according to the present disclosure may include (a) adding a synthesis material and a solvent to a precursor mixing chamber to which an injection pipe is connected and performing vortexing, and (b) moving the solution prepared above to a reaction chamber connected to the mixing chamber and allowing gas to flow at a high speed on the boundary of the reaction chamber and in a liquid cutting tube with a gap ranging from tens of nanometers to hundreds of micrometers therebetween.

In one embodiment, the method may further include (c) collecting the cut solution from the boundary of the reaction chamber and the liquid cutting tube and collecting the same again in a predetermined amount at the boundary of the reaction chamber, and separating or extracting the synthesized reaction product from the boundary and performing circulation to bind a target material in a binding chamber.

In one embodiment, the side angle of the inlet of the liquid cutting tube may be 5 degrees to 60 degrees, and the front angle may be 5 degrees to 60 degrees.

In one embodiment, the reaction chamber may have a structure with an inlet and an outlet or a structure that is simple to open and close to allow efficient air flow.

In one embodiment, the binding chamber may be configured such that a tube with an annular structure or a continuous zigzag structure is connected to a circulator to enable circulation of the reaction product (nanoparticles or a drug) prepared in step (c).

In one embodiment, the nanoparticles manufactured by the manufacturing method described above may be a diagnostic or therapeutic nanomedicine, configured such that any one or more selected from among a photosensitizer, a first-generation anticancer agent, a second-generation anticancer agent, a third-generation anticancer agent, a nuclear medicine therapeutic drug, a metabolic anticancer agent, an enzyme, a gene therapeutic agent, and a near-infrared fluorescent dye are bound.

In one embodiment, the drug manufactured by the manufacturing method described above may be synthesized at a low temperature that is not the freezing point of the solvent used or at a high temperature that does not evaporate all of the solvent, and may be synthesized in a short time with a small volume compared to drugs manufactured without the above device.

In one embodiment, the biomedicine is configured such that the drug manufactured by the manufacturing method described above is bound to any one or more selected from the group consisting of a nucleic acid-based molecule, an amino acid-based antibody, a specific binding protein, and an enzyme with high biosafety.

**Below is a description of various aspects of the present disclosure.**

An aspect of the present disclosure provides a method of manufacturing nanoparticles or a nanomedicine, including (a) adding a synthesis material and a solvent to a precursor mixing chamber to which an injection pipe is connected and performing vortexing, (b-1) moving the solution prepared in step (a) to a reaction chamber 30 connected to the mixing chamber, and (b-2) allowing gas to flow at a high speed on the boundary of the reaction chamber 30 and in the liquid cutting tube 31 with a gap ranging from 10 nanometers to 1,000 micrometers therebetween.

In an aspect of the present disclosure, the method further includes (c) collecting the cut solution from the boundary of the reaction chamber 30 and the liquid cutting tube 31 and collecting the same again in a predetermined amount at the boundary of the reaction chamber.

In an aspect of the present disclosure, the method further includes (d) separating or extracting the synthesized reaction product from the boundary and performing circulation to bind a target material in a separate binding chamber.

In the method according to an aspect of the present disclosure, the side angle of the inlet of the liquid cutting tube 31 is 5 degrees to 60 degrees, and the front angle is 5 degrees to 60 degrees.

In the method according to an aspect of the present disclosure, in step (b-2), the gas is designed to flow at a high speed due to voltage, and the gas is allowed to flow at a high speed at an applied voltage of 5 to 60 kV.

In the method according to an aspect of the present disclosure, the boundary of the reaction chamber and the liquid cutting tube are at 0.25 to 0.85 rad.

In the method according to an aspect of the present disclosure, the gap in step (b-2) falls in the range of 500 nm to 1500 nm.

In the method according to an aspect of the present disclosure, the nanoparticles or the nanomedicine are a photosensitizer, a first-generation anticancer agent, a second-generation anticancer agent, a third-generation anticancer agent, a nuclear medicine therapeutic drug, a metabolic anticancer agent, an enzyme, a gene therapeutic agent, or a near-infrared fluorescent dye.

Another aspect of the present disclosure provides nanoparticles or a nanomedicine manufactured by the method according to any one aspect of the present disclosure described above.

### [Mode for Invention]

A better understanding of the present disclosure may be obtained through the following examples. These examples are merely set forth to illustrate the present disclosure, and are not to be construed as limiting the scope of the present disclosure.

### Example 1. Manufacture of Berlin blue nanoparticles and nanomedicine

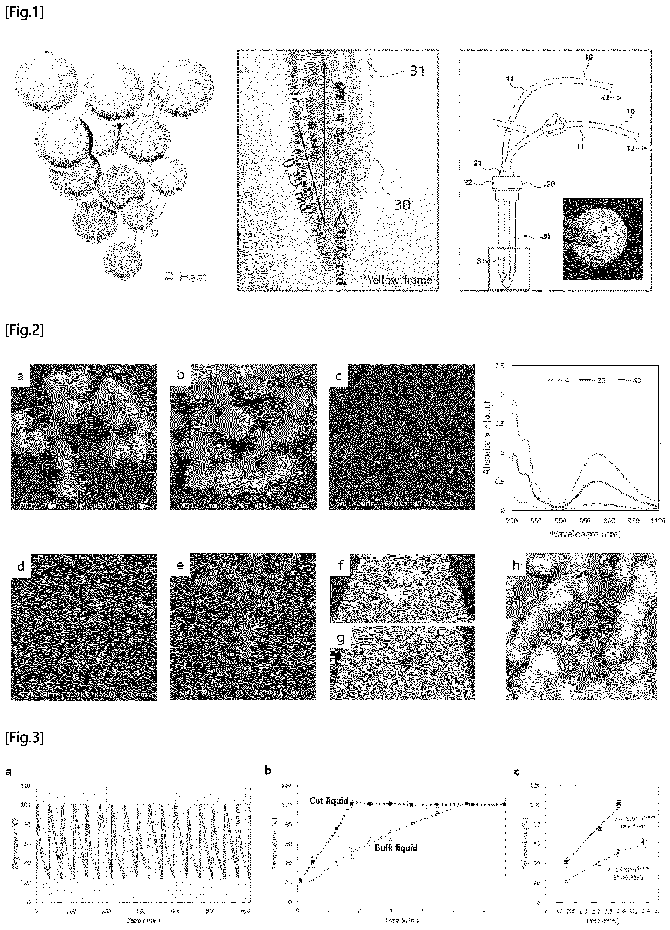

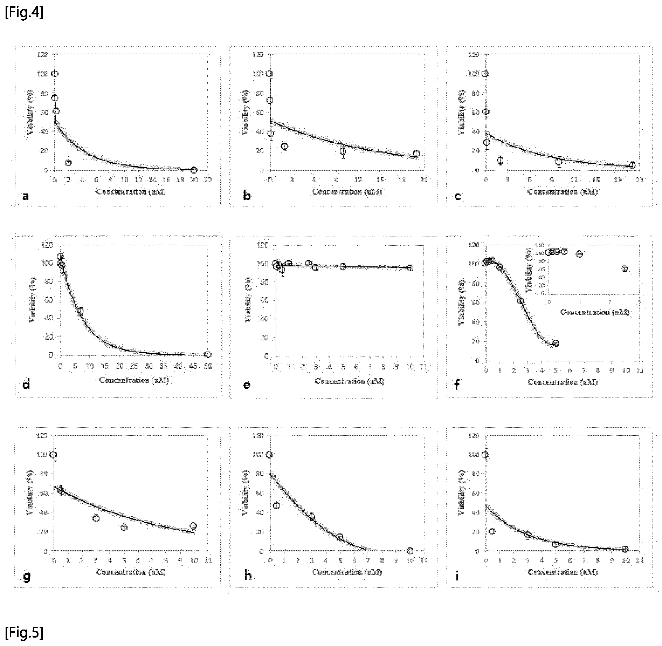

1 g of polyvinylpyrrolidone (PVP, Sigma-Aldrich) was dissolved in 20 g of ultrapure water and stirred in a mixing chamber at 1500 RPM for 30 minutes to obtain an aqueous PVP solution. Subsequently, 131.7 mg of potassium hexacyanoferrate(III) trihydrate (K₃Fe(CN)₆·3H₂O, Sigma-Aldrich) was dissolved in 20 g of ultrapure water and mixed with the aqueous PVP solution. Then, 400 µl of 1 M HCl (Sigma-Aldrich) was added to the mixing chamber and vortexed at 60 J/s. After movement through an injection pipe, the solution thus prepared was subjected to a step of allowing gas to flow at a high speed on the inner boundary of a reaction chamber and in a liquid cutting tube at an applied voltage of 30 kV, a distance of 25 cm between the reaction chamber and the end of the injection pipe, and a temperature of 140°C. As indicated by the green arrows in FIG. 1, the gas is moved toward the upper portion of the reaction chamber through a structure in which the boundary of the reaction chamber and the liquid cutting tube are at 0.29 rad and the gap of the boundary is 900 nm. As the gas passed, the prepared solution was cut into small pieces. The cut liquid received heat in the reaction chamber and the temperature thereof rose quickly compared to bulk liquid having the same total volume (40 ml) due to heat transfer within the liquid (FIG. 3). Simultaneously with reaction of the solution in the reaction chamber, the cut solution was collected from the boundary of the reaction chamber and the liquid cutting tube, and was collected again in a predetermined amount at the boundary of the reaction chamber. After 3 hours, when reaction was completed, the synthesized reaction product was extracted through centrifugation from the boundary and washed with water. Thereafter, the washed result was moved to a binding chamber through pumping and circulated to bind 2 mg of docetaxel (DTX) or pyropheophorbide-a methyl ester (MPPa) in the binding chamber. The completed sample was washed three times with methanol and distilled water, dried, and manufactured into a nanomedicine with a diameter of 200 nm. In order to confirm the efficiency of killing cancer cells, cells cultured in a 96-well plate for 24 hours were treated with nanodrugs at various concentrations. The photothermal effect of the drugs was confirmed through CCK8 assay by irradiating the target with lasers at 808 nm for 2 minutes at a voltage of 9.26 V corresponding to 1 W/cm^2 and a distance of 8.54 cm between the target and the laser, in which the diameter of the spot area (optical fiber itself) was 3.5 cm. The photodynamic therapy effect of the drugs was confirmed through CCK8 assay by irradiating the target with lasers at 660 nm (ThorLabs ED1-C50-MD, Ø1.0" 50° circle, equipped with TOP_HAT diffuser) for 10 minutes at a voltage of 0.44 V corresponding to 1-2.3 mW/cm^2 and a distance of 16 cm between the target and the laser, in which the spot diameter was 24 cm. Based on the results of CCK8 assay, the effects of anticancer agents were also confirmed in the nanodrugs, and additionally, the effects of photothermal therapy (PTT) and photodynamic therapy (PDT) were also exhibited (FIG. 4).

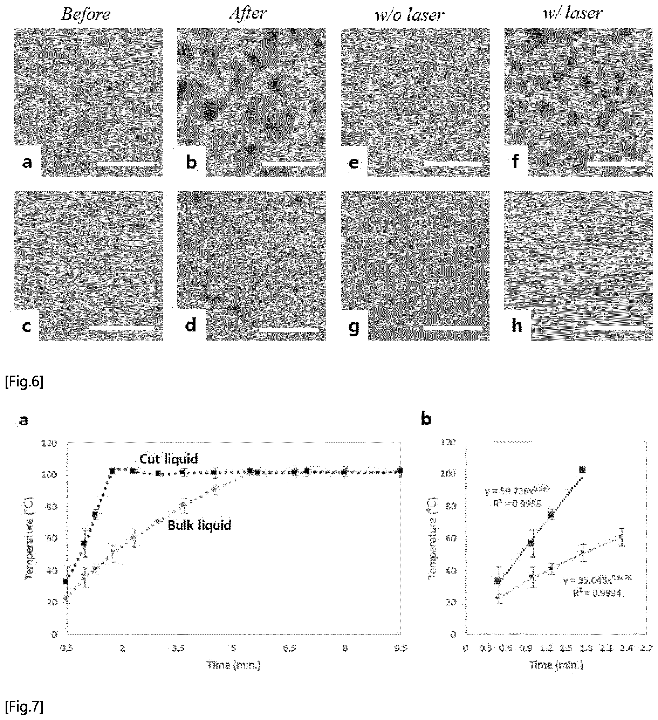

FIG. 2 shows morphologies of the Berlin blue nanoparticles (a) according to the manufacturing method of the present disclosure and the nanomedicine (b) of MPPa-bound Berlin blue nanoparticles, FIG. 3 shows results of the time taken for the mixed solution to reach the boiling point, and FIG. 4 shows the effects of killing cancer cells (PANC-1) (a = docetaxel treatment; b = mPB-docetaxel treatment; c = mPB-docetaxel and PTT treatment; d = MPPa treatment; e = mPB-MPPa treatment; f = mPB-MPPa treatment and PDT treatment; g = mPB-MPPa treatment and PTT treatment; h = mPB-MPPa treatment and PDT followed by PTT treatment; i = mPB-MPPa treatment and PTT followed by PDT treatment) . Moreover, as shown in FIG. 5, cellular uptake of the nanodrugs into cancer cells was confirmed (a, b), cancer cells were killed by anticancer agent (DTX)-bound Berlin blue nanoparticles (c, d), and the PDT effect (e, f) and the PTT effect (g, h), in which cancer cells were killed by MPPa-bound Berlin blue nanoparticles, were also confirmed (scale bar = 50 nm). Based on these results, the manufacturing method of the present disclosure was proven to be a method capable of manufacturing Berlin blue nanoparticles, reducing the time to reach the high temperature compared to when manufacturing nanoparticles from bulk liquid having the same weight, and manufacturing a finished product with the function of a nanomedicine.

### Example 2. Manufacture of ceria nanoparticles

A 0.3 mol/L cerium(IV) diammonium nitrate solution and a 0.6 mol/L ammonium sulfate solution were added to a mixing chamber and vortexed at 40 J/s. After movement through an injection pipe, the solution thus prepared was subjected to a step of allowing gas to flow at a high speed on the inner boundary of a reaction chamber and in a liquid cutting tube at an applied voltage of 30 kV, a distance of 25 cm between the reaction chamber and the end of the injection pipe, and a temperature of 230°C. Simultaneously with reaction of the solution in the reaction chamber, the cut solution was collected from the boundary of the reaction chamber and the liquid cutting tube, and was collected again in a predetermined amount at the boundary of the reaction chamber. After 1 hour, when the reaction was completed, the synthesized reaction product was extracted through centrifugation from the boundary and washed with water.

FIG. 2 shows the morphology of ceria nanoparticles (c). The previously developed method including reaction at 91°C for 3 hours required additional drying at a high temperature and thermal treatment at 800°C of 50 nm-sized small particles, which has the problem of making it difficult to obtain uniform and independent particles. However, according to the manufacturing method of the present disclosure, the nanoparticles had an average size of 160 nm and were uniform, and the reaction time was shorter based on a total volume of 100 ml (FIG. 6).

### Example 3. Manufacture of gold nanoparticles

20 ml of an aqueous 1 mM HAuCl₄ solution was placed in a mixing chamber, moved to a reaction chamber through an injection pipe, and then subjected to a step of allowing gas to flow at a high speed on the inner boundary of the reaction chamber and in a liquid cutting tube at an applied voltage of 30 kV, a distance of 5 cm between the reaction chamber and the end of the injection pipe, and a temperature of 110°C. Subsequently, 2 ml of an aqueous 1% sodium citrate solution was added to the mixing chamber, and the aqueous solution was moved and added to the 1 mM HAuCl₄ solution in the reaction chamber. Simultaneously with reaction of the mixed solution in the reaction chamber, the cut solution was collected from the boundary of the reaction chamber and the liquid cutting tube, and was collected again in a predetermined amount at the boundary of the reaction chamber. After 10 minutes, when the reaction was completed, the synthesized gold nanoparticles were separated from the boundary and purified.

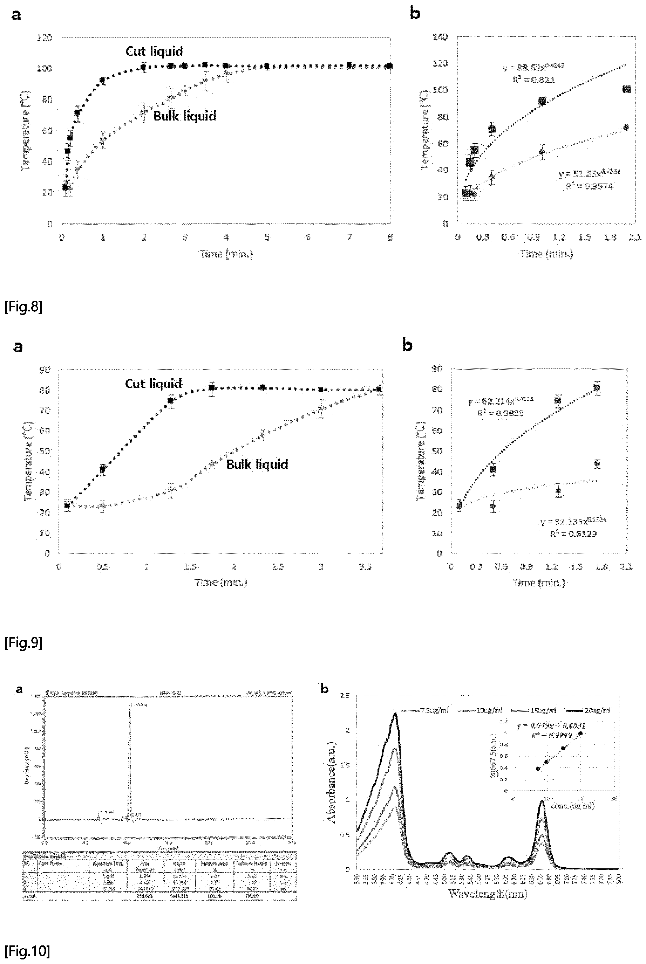

FIG. 2 shows the morphology of gold nanoparticles (d). In existing wet chemical synthesis methods, it was difficult to obtain, as gold nanoparticles having a size of about 200 nm, uniform and independent particles, but the nanoparticles obtained by the manufacturing method of the present disclosure were uniform with an average size of 180 nm. Therefore, the separation process may be omitted. In addition, when comparing the transfer rates of increased temperature, the manufacturing method of the present disclosure can be found to reduce the reaction time (FIG. 7).

### Example 4. Manufacture of palladium metal nanoparticles

5 g of polymethyl methacrylate (PMMA, Sigma-Aldrich) was dissolved in 40 g of DMF to obtain a PMMA solution. Then, this solution was added to 0.5 g of palladium dichloride (PdCl₂) in a mixing chamber and stirred at 600 RPM for 30 minutes. After movement through an injection pipe, the solution thus prepared was subjected to a step of allowing gas to flow at a high speed on the inner boundary of a reaction chamber and in a liquid cutting tube at an applied voltage of 30 kV, a distance of 25 cm between the reaction chamber and the end of the injection pipe, and a temperature of 80°C. Simultaneously with reaction of the solution in the reaction chamber, the cut solution was collected from the boundary of the reaction chamber and the liquid cutting tube, and was collected again in a predetermined amount at the boundary of the reaction chamber. After 2 hours, the reaction was terminated and the reaction product was transferred to an alumina (Al₂O₃) crucible and treated at 400°C for 1 hour, yielding palladium metal nanoparticles.

FIG. 2 shows a scanning electron microscope image of palladium metal nanoparticles (e). As shown in the drawing, it was confirmed that the palladium metal nanoparticles were very uniformly distributed with an average size of 205 nm. In addition, when comparing the transfer rates of increased temperature, the manufacturing method of the present disclosure can be found to reduce the reaction time (FIG. 8).

### Example 5. Manufacture of pyropheophorbide-a methyl ester (MPPa)

All glassware used was washed with acetone and then dried or used after drying at 60°C. First, as a control, a general method of synthesizing pyropheophorbide-a methyl ester (MPPa) was carried out as follows. 2 N hydrochloric acid was prepared by adding deionized (D.I.) water to 173 mL of 35% hydrochloric acid so that a final volume was 1 L. Then, 1 g of methyl pheophorbide-a (MPa) and 100 ml of 2,4,6-trimethylpyridine were mixed and stirred at 175°C for 3 hours. After washing with equal amounts of 2 N hydrochloric acid and dichloromethane, the organic solvent layer was collected and distilled under reduced pressure. Subsequently, for purification, the inlet of a column was blocked using a small amount of cotton, and sea sand was poured over the cotton to fill the curved surface at the bottom of the column. Then, a mixture in a liquid phase including silica powder and hexane in a 5 L tall beaker was added to 2/3 of the height of the column. Thereafter, when the height of hexane reached the same level as the height of silica, the compound dissolved in the solvent was slowly loaded along the wall surface of the column. Then, sea sand was added to the height of the compound layer, followed by purification using a mobile-phase solvent (acetone:dichloromethane = 2:98) and then distillation under reduced pressure to obtain a powder.

Next, synthesis of MPPa using the manufacturing method of the present disclosure was as follows. 1 g of MPa and 100 ml of 2,4,6-trimethylpyridine were added to a mixing chamber and vortexed at 20 J/s. After movement through an injection pipe, the solution thus prepared was subjected to a step of allowing gas to flow at a high speed on the inner boundary of a reaction chamber and in a liquid cutting tube at an applied voltage of 30 kV, a distance of 25 cm between the reaction chamber and the end of the injection pipe, and a temperature of 175°C. Simultaneously with reaction of the solution in the reaction chamber, the cut solution was collected from the boundary of the reaction chamber and the liquid cutting tube, and was collected again in a predetermined amount at the boundary of the reaction chamber. After 1 hour, when the reaction was completed, the synthesized reaction product at the boundary was washed with equal amounts of 2 N hydrochloric acid and dichloromethane, and the organic solvent layer was collected and distilled under reduced pressure. Subsequently, column chromatography for purification was performed in the same manner as above. The purified MPPa was analyzed by ¹H-NMR using tetramethylsilane (TMS), which is inert to the sample and the organic solvent, as a reference material, and absorbance was measured by dissolving the powder in dichloromethane. In addition, based on the results of HPLC analysis of MPPa, the purity was 95.4% (FIG. 9a).

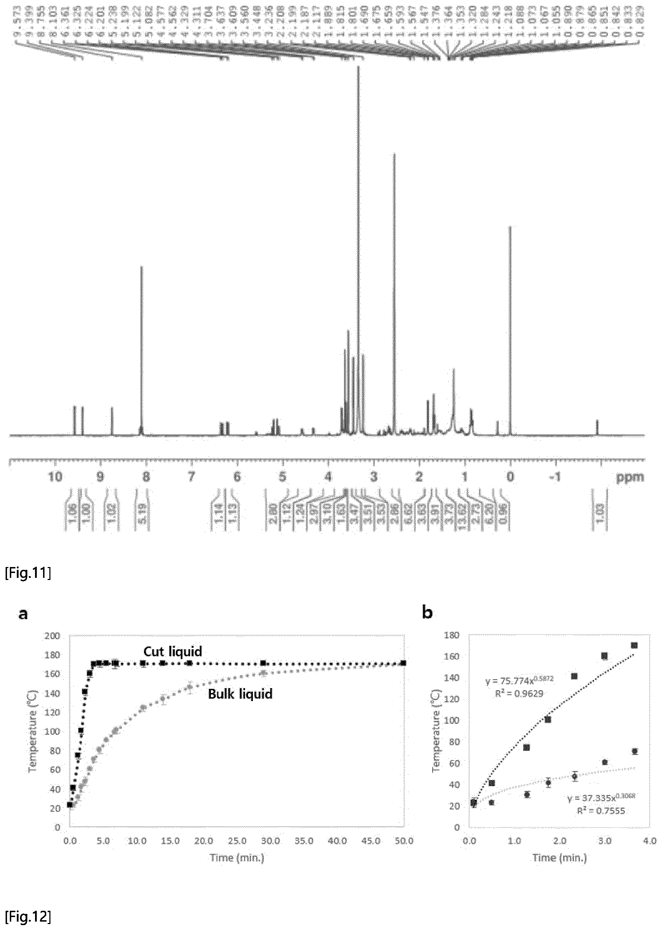

FIG. 9 shows UV-Vis spectra of MPPa synthesized by the manufacturing method of the present disclosure (FIG. 9b). Xmax (CH₂Cl₂)/nm 667 (rel. intensity 0.457), 610 (0.078), 540 (0.092), 508.5 (0.106), 413.5 (1.108); it can be seen that MPPa was synthesized based on the clear appearance of five major peaks and the positions of NMR peaks (FIG. 10). ¹H-NMR (500 MHz, DMSO, TMSᵢₙₜ) δ_{H}, ppm 9.57 (1 H, s, 5-H), 9.39 (1 H, s, 10-H), 8.75 (1 H, s, 20-H), 8.10 (1 H, m, 3¹-CH), 6.34 and 6.21 (each 1 H, dd, 3²-CH₂), 5.15 (2 H, q, 13²-CH₂), 4.56 (1 H, m, 18-CH), 4.31 (1 H, m, 17-CH),3.63 (3 H, s, 17⁴-OCH₃), 3.56 (3 H, s, 12¹-CH₃), 3.44 (3 H, s, 2¹-CH₃), 3.23 (3 H, s, 7¹-CH₃), 2.77-2.62 (2 H, m, 17¹-CH₂), 2.47-2.31 (2 H. m, 17²-CH₂), 1.81 (3 H, d, 18¹-CH₃), 1.67 (3 H, t, 8²-CH₃), 0.28 and - 1.91 (each 1 H, br, s, 2 X NH). In addition, when comparing a conventional bulk method with a liquid cutting method, the manufacturing method of the present disclosure can be found to start the reaction more quickly (FIG. 11).

### Example 6. Manufacture of paclitaxel analogues

Docetaxel (DTX) is a taxane-based anticancer agent that is used to treat breast cancer, head and neck cancer, stomach cancer, prostate cancer, and non-small cell lung cancer and may be used alone or in combination with other chemotherapy drugs. Referring to Korean Patent Application Publication No. 10-2007-0062533, it was applied to novel technology of the present disclosure. To summarize the manufacturing method, first, 16 g of compound (I) prepared was dissolved in a 1:1 mixture (320 ml) of absolute alcohol (EtOH) and methylene chloride (DCM) in a mixing chamber, and 5.27 g (in 5 ml of DCM) of di-tert-butyl dicarbonate (Boc₂O) was added and vortexed at 20 J/s. 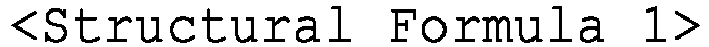 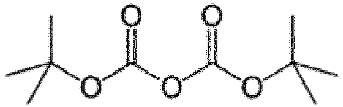 Di-tert butyl dicarbonate

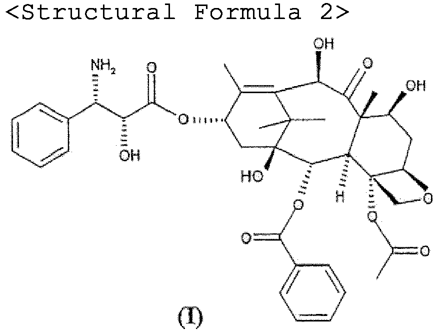

The reaction mixture was then stirred at room temperature for 16 hours. DCM was removed by distillation under reduced pressure, and 0.39 ml of acetic acid was added to the solution. The acidic ethanol solution was heated to 50°C and 320 ml of ultrapure water was added dropwise. After movement through an injection pipe, the solution thus prepared was subjected to a step of allowing gas to flow at a high speed on the inner boundary of a reaction chamber and in a liquid cutting tube at an applied voltage of 30 kV, a distance of 25 cm between the reaction chamber and the end of the injection pipe, and a temperature of 50°C. Simultaneously with reaction of the solution for 10 minutes in the reaction chamber, the cut solution was collected from the boundary of the reaction chamber and the liquid cutting tube, and was collected again in a predetermined amount at the boundary of the reaction chamber and then left at room temperature for an additional 2 hours. The precipitate was filtered through a sintered glass filter, then transferred into a vacuum oven and maintained in a vacuum at 40°C for 16 hours, yielding 16.75 g of semi-purified docetaxel.

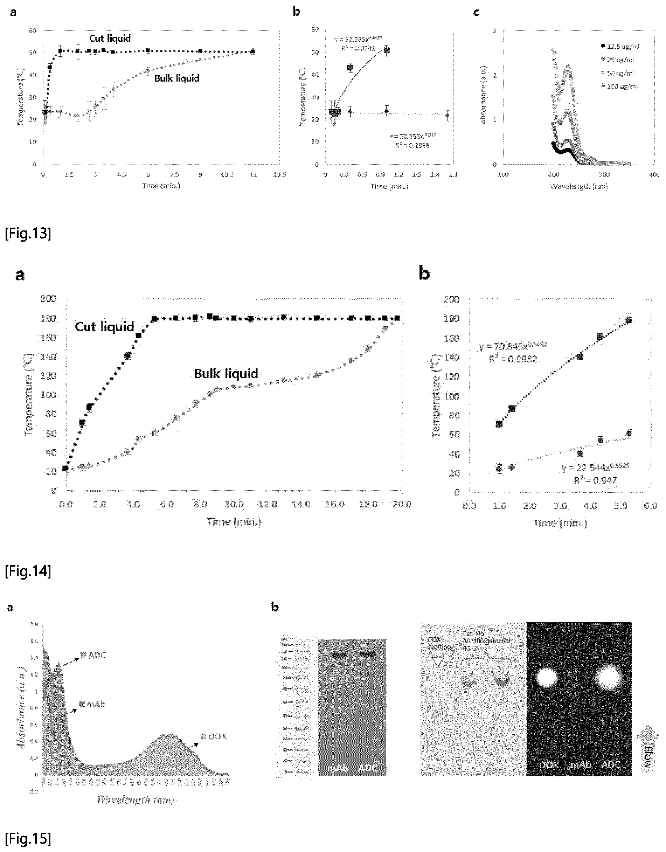

Thereafter, semi-purified docetaxel was dissolved in 95% ethanol (160 ml) at 50°C and acetic acid (0.39 ml) was added. Ultrapure water (320 ml) was added, and after movement through the injection pipe, the solution thus prepared was subjected to a step of allowing gas to flow at a high speed on the inner boundary of the reaction chamber and in the liquid cutting tube at an applied voltage of 30 kV, a distance of 25 cm between the reaction chamber and the end of the injection pipe, and a temperature of 50°C. Simultaneously with reaction of the solution for 10 minutes in the reaction chamber, the cut solution was collected from the boundary of the reaction chamber and the liquid cutting tube, and was collected again in a predetermined amount at the boundary of the reaction chamber and then left at room temperature for an additional 2 hours. The precipitate was filtered through a sintered glass filter and maintained in a vacuum at 40°C for 16 hours to obtain 15.25 g of docetaxel. The second crystallization was performed by mixing the product dissolved in acetone (150 ml) with 150 ml of heptane at 30°C. The mixture was left at room temperature for 3 hours, filtered through a sintered glass filter, and dried in a vacuum at 40°C for 16 hours. Finally, 13.9 g of docetaxel was obtained (HPLC confirmation: purity higher than 99.4%, <0.1% 7-epi docetaxel), <0.1% 10-dehydrodocetaxel), synthesis of which was confirmed through UV spectra (FIG. 12c). 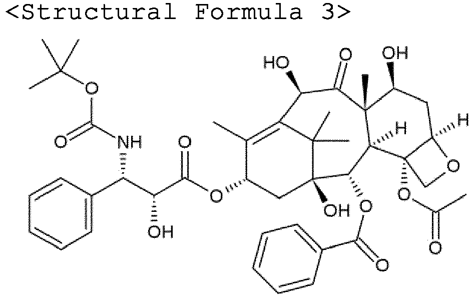 Docetaxel

As can be seen in FIG. 4a, docetaxel synthesized by the manufacturing method of the present disclosure increased the efficiency of killing cancer cells with an increase in the treatment concentration. This is the result of docetaxel binding to beta-tubulin of microtubules. Furthermore, in the way of synthesis, when comparing a conventional bulk method with a liquid cutting method, the manufacturing method of the present disclosure can be found to start the reaction more quickly (FIG. 12).

### Example 7. Melting test of hypromellose for tablet dosage form

1 g of hydroxypropyl methylcellulose (H8384, SA), either alone or in combination with 100 mg of MPPa prepared using the manufacturing method of the present disclosure, was added to a mixing chamber and vortexed at 90 J/s with 20 g of 50% DMSO solution. After movement through an injection pipe, the mixed solution was subjected to a step of allowing gas to flow at a high speed on the inner boundary of a reaction chamber and in a liquid cutting tube at an applied voltage of 10 kV, a distance of 25 cm between the reaction chamber and the end of the injection pipe, and a temperature of 178.9°C. Simultaneously with reaction of the solution in the reaction chamber, the cut solution was collected from the boundary of the reaction chamber and the liquid cutting tube, and was collected again in a predetermined amount at the boundary of the reaction chamber. After 1 hour, when the reaction was completed, the melted reaction product was taken out from the boundary and filtered with ultrapure water. Thereafter, the reaction product was dried for 24 hours to obtain a paste, which was then stored. Some stored hydroxypropyl methylcellulose granules or MPPa-mixed granules were extruded at 50°C and compressed into tablets (FIG. 2f).

In addition, for the manufacture of rifaximin tablets, a mixture including 200 mg of rifaximin, 30 mg of sodium starch glycolate (type A), 10 mg of disodium edetate hydrate, and 12 mg of hydroxypropyl methylcellulose 2910 (viscosity of about 4,000 cP, 2% in H₂O (20°C) (lit.), SA) (or MPPa-mixed granules above) was placed in a high-shear mixing granulator and mixed for 5 minutes. Thereafter, 213 mg of ultrapure water was added and mixed for 15 minutes. For drying, the reaction mixture was left at 40°C for 24 hours and sieved through a 2 mm screen. Then, the dried granules were transferred to a mixer (Servolift bin blender), and 15 mg of colloidal silica (anhydrous) and 4 mg of stearic acid were added together. The mixer was set to 6 RPM and mixing was terminated after 20 minutes. The granules were then transferred to a tablet press (Korsch EK0 tablet press) for compression. The obtained slug was then sieved through a 1.6 mm screen. The sieved slug was transferred to a mixer (Servolift bin blender), 4 mg of stearic acid was added to the mixer, the mixer was set to 6 RPM, and mixing was terminated after 20 minutes. The slug was then transferred to a tablet press (Korsch PH 106 tablet press) and compressed at 20 kN pressure to obtain rifaximin tablets. Additionally, rifaximin tablets were placed in a coating machine (BYC-400), 12 mg of hydroxypropyl methylcellulose (H8384, SA), 1.2 mg of polyethylene glycol 6000, 0.8 mg of titanium oxide, 1 mg of talc, and 100 mg of ultrapure water were added, and the inlet air was set to 50°C, followed by coating for 70 minutes (FIG. 2g).

The melting point of hydroxypropyl methylcellulose (viscosity of 40-60 cP, 2% in H₂O (20°C) (lit.), H8384, SA) is 178.9°C. In the mixed solution including hydroxypropyl methylcellulose, as the concentration of hydroxypropyl methylcellulose increases, viscosity also increases, making melting difficult. As such, increasing the temperature makes melting easier. As shown in FIG. 13, there was a difference between the melting rate in the bulk solution and the melting rate in the cut liquid based on a difference in temperature rise. As such, the cut liquid reached the target temperature more quickly due to a difference in surface area, indicating that the method of the present disclosure was capable of reducing the time to melt hydroxypropyl methylcellulose compared to the control.

### Example 8. Production of antibody-drug conjugate

First, antibody reduction-alkylation proceeded as follows. Panitumumab (from Vectibix) was buffered with 25 mM sodium borate at pH 8, 25 mM NaCl, 5 mM EDTA, and 10 mM TCEP to have a concentration of 10 mg/ml, 8 mol of TCEP was prepared per mol of antibody, and 10 mM TCEP was added, followed by stirring at 40°C for 2 hours. Removal of unreacted materials was performed through filtration using a PD-10 column in ice cold 40/60 (% v/v) DMSO/0.1 M Tris-HCl at pH 8 and 1 mM EDTA (Tris/DMSO buffer). The antibody was then condensed using a 30 kDa MWCO Amicon filter.

Next, a linker-anticancer agent was prepared as follows. In the mixing chamber of the present disclosure, 29.4 g of maleic anhydride and 39.35 g of 6-aminocaproic acid were allowed to react in 900 ml of glacial acetic acid for 16 hours. Then, 30.6 g of acetic anhydride was added dropwise for 2 hours and stirred for 1 hour. For subsequent solvent removal reaction, after movement through an injection pipe, the solution was subjected to a step of allowing gas to flow at a high speed on the inner boundary of a reaction chamber and in a liquid cutting tube at an applied voltage of 30 kV, a distance of 25 cm between the reaction chamber and the end of the injection pipe, and a temperature of 70°C. After 20 minutes, the solid was collected from the reaction chamber and dried for 2 hours. 2.11 g of dried 6-maleimidohexanoic acid was dissolved in 200 ml of tetrahydrofuran, and 1 g of N-methylmorpholine was added and stirred in the mixing chamber. Thereafter, the mixture was transferred to the reaction chamber and 1.36 g of an isobutyl chloroformate solution was added dropwise. Additionally, 1.32 g of tert-butyl carbazate was added dropwise, and the reaction mixture was allowed to react at 4°C for 30 minutes and then allowed to stand at room temperature for 1 hour. The organic layer was removed by washing and then drying over anhydrous sodium sulfate. 545 mg of the finished product was dissolved in 10 ml of cold trifluoroacetic acid and stirred in an ice bath for 8 minutes, and the produced acid was removed. Thereafter, column chromatography was performed using methylene chloride-methanol-ammonium hydroxide (100:5:0.5) solvents, followed by drying. After mixing 9.2 g of dried 6-maleimidocaprohydrazide and 5.2 g of doxorubicin hydrochloride with 1750 ml of methanol, 0.5 ml of trifluoroacetic acid was added, followed by stirring at room temperature for 24 hours. Then, at 31°C, only 250 ml was left behind and 1250 ml of acetonitrile was added. After waiting at 4°C for 48 hours, the result was separated by centrifugation, washed with methanol-acetonitrile, and then dried.

The antibody and the linker-anticancer agent (MC-DOXHZN), prepared as above, were conjugated through the following process. 10 mg of the antibody was mixed with 23 ml of 10 mM DTT and allowed to react for 3 hours. The remaining DTT was removed by centrifugal dialysis, followed by reaction with an equal amount of 1 mol MC-DOXHZN at 4°C for 30 minutes. Thereafter, the reaction result was filtered through a cellulose acetate membrane and washed using a Bio-Rad BioBeads SM-2 Resin column. Finally, 9.2 mg/ml of the conjugate was obtained.

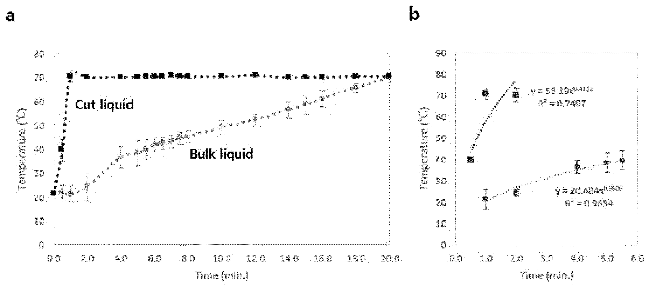

FIG. 14 shows results of UV-Vis spectra and dot blot analysis of the antibody-drug conjugate (ADC) synthesized by the manufacturing method of the present disclosure. The sample was subjected to non-reducing blotting without DTT or beta-mercaptoethanol to confirm the presence or absence of conjugation. Thereby, the fluorescence of DOX appeared in the antibody-drug conjugate and changes in UV-Vis spectra were confirmed, indicating production of ADC (ADC = antibody-drug conjugate, DOX = doxorubicin, mAb = panitumumab). In view of synthesis time, when comparing a conventional bulk method with a liquid cutting method, the manufacturing method of the present disclosure can be found to start the reaction more quickly (FIG. 15).

Although specific embodiments of the present disclosure have been described, those skilled in the art will appreciate that the present disclosure may be embodied in other specific forms without changing the technical spirit or essential features thereof. Thus, the embodiments described above should be understood to be non-limiting and illustrative in every way.

### [Description of the Reference Numerals in the Drawings]

- 10:: mixture inlet
- 11:: injection pipe
- 12:: connector of mixing chamber
- 20:: air circulator
- 21:: air inlet
- 22:: air outlet
- 30:: reaction chamber
- 31:: liquid cutting tube
- 40:: product outlet
- 41:: discharge pipe
- 42:: connector of binding chamber

## Claims

1. A method of manufacturing a medicine, comprising:
(a) adding a synthesis material and a solvent to a precursor mixing chamber to which an injection pipe is connected and performing vortexing;
(b-1) moving a solution prepared in step (a) to a reaction chamber connected to the mixing chamber; and
(b-2) allowing a gas to flow at a high speed on a boundary of the reaction chamber and in a liquid cutting tube with a gap ranging from 10 nanometers to 1,000 micrometers therebetween.

2. The method of claim 1, further comprising (c) collecting a cut solution from the boundary of the reaction chamber and the liquid cutting tube and collecting the cut solution again in a predetermined amount at the boundary of the reaction chamber.

3. The method of claim 2, further comprising (d) separating or extracting a synthesized reaction product from the boundary and performing circulation to bind a target material in a separate binding chamber.

4. The method of claim 1, wherein a side angle of an inlet of the liquid cutting tube is 5 degrees to 60 degrees, and a front angle is 5 degrees to 60 degrees.

5. The method of claim 1, wherein, in step (b-2), the gas is designed to flow at a high speed due to a voltage, and the gas flows at a high speed at an applied voltage of 5 to 60 kV.

6. The method of claim 1, wherein the medicine is nanoparticles, an organic compound, an inorganic compound, or a nanomedicine for diagnosis or treatment, and the nanoparticles or the nanomedicine are configured such that any one or more selected from among a photosensitizer, a first-generation anticancer agent, a second-generation anticancer agent, a third-generation anticancer agent, a nuclear medicine therapeutic drug, a metabolic anticancer agent, an enzyme, a gene therapeutic agent, and a near-infrared fluorescent dye are bound.

7. The method of claim 1, wherein the reaction chamber has a structure with an inlet and an outlet or a structure that is simple to open and close to allow efficient air flow.

8. The method of claim 3, wherein the binding chamber is configured such that a tube with an annular structure or a continuous zigzag structure is connected to a circulator to enable circulation of the reaction product (nanoparticles or a drug) obtained in step (c).

9. A medicine manufactured by the method of any one of claims 1 to 8.

10. The medicine of claim 9, wherein the medicine is synthesized at a low temperature that is not a freezing point of a solvent used or at a high temperature that does not evaporate all of the solvent, and is synthesized in a short time with a small volume compared to drugs manufactured by different methods.

11. A biomedicine configured such that the medicine of claim 9 is bound to at least one selected from the group consisting of a nucleic acid-based molecule, an amino acid-based antibody, a specific binding protein, and an enzyme with high biosafety.
